# EUROPEAN PATENT SPECIFICATION

(11) **EP 1 257 324 B1**
(45) Date of publication and mention of the grant of the patent: **13.01.2010**
(21) Application number: 00913974.2
(22) Date of filing: 11.04.2000
(51) Int. Cl.: A61N 5/067, A61N 5/06

(54) **IMPROVED LASER COMB DESIGN/FUNCTION**
VERBESSERTE DESIGN/FUNKTION EINES LASERKAMMES
CONCEPTION/FONCTION AMELIOREES D'UN PEIGNE A LASER

(30) Priority: 17.02.2000 AU 1754800
(43) Date of publication of application: 20.11.2002
(73) Proprietor: Lexington LaserComb IP AG, 9000 St. Gallen (CH)
(72) Inventor: Pearl, Henry, Willoughby, NSW 2066 (AU)
(74) Representative: Findlay, Alice Rosemary
(86) International application number: PCT/AU2000/000302
(87) International publication number: WO 2001/060457

(56) References cited:
- EP-A- 0 593 375
- DE-A- 3 336 939
- DE-A- 3 511 281
- DE-U- 9 102 407
- FR-A- 2 518 412
- US-A- 5 300 097
- US-A- 5 303 722

## Description

A hand-held, comb-like device emitting laser beams from laser diodes set in a row centered between two parallel rows of teeth for parting hair, enabling more economical product manufacturing as well as a more effective way of irradiating hair-growing skin with laser energy than that afforded by conventional hand-held laser devices where the laser energy is conveyed via a system of fiber optics into and through tubes which must also act as teeth for parting hair.

DE-U-91 02 407 discloses a device or the above described conventional design

US-A-2397757 discloses a hair brush with a UV ray generating source within a cavity, a brush mount enclosing the source within the cavity bristles secured to the mount and teeth integral with or secured to the mount. The mount may be made of a material which does not transmit UV rays and the bristles and teeth of a material which transmits UV rays.

DE-A-3336939 discloses a device with a tubular UV lamp which emits UV light through an opening in a housing. The housing is shaped or the edges of the opening to form teeth.

Low-power laser is in widespread use in skin care, scar reduction, wound healing and the like, and has also been found to assist the hair's natural ongoing replacement and improve its condition. However, when there is existing hair growth on the skin being treated (for whatever purpose), laser beams by themselves do not satisfactorily penetrate the hair and it is necessary to find means to enable the laser beam or beams to be better brought into contact with the hair-growing skin.

In this invention as defined by claim 1 laser energy reaches the hair-growing skin by way of a row of laser beams being preceded by a row of teeth that part the hair to expose the skin in advance of said row of laser beams. Said laser irradiation is thus direct in its action instead of as is done via the unnecessarily complex conventional system which uses tubes, each one containing a fiber optic to both carry the laser light and part the hair, requiring said tubes to be broader than the teeth of this invention which are solid and can be finer and more sharply pointed and plow-like in shape and function, affording more effective hair parting than that of aforesaid conventional system.

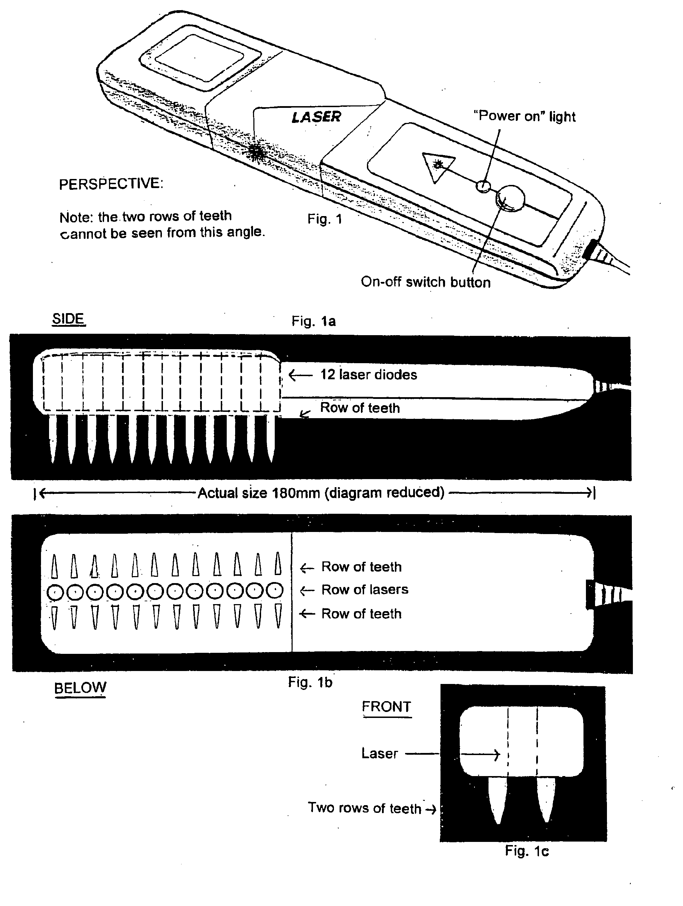

This invention will now be described with reference to the accompanying drawings which show internal details which are encased in the device. (See fig. 1, fig. 1a, fig. 1b, fig. 1c.)

In this device solid state diode lasers are used. The diodes usually come from the manufacturer within complete modules which are small cylindrical units each approximately 20mm long by 6mm in diameter in which is encased the losing medium which creates the laser beam and the electronics that control and process the electric current that passes through the lasing medium.

The lasing medium chosen for use in the preferred form of this invention produces a visible red beam of between 632 to 670 nanometers and has a typical divergence of 33 degrees in the perpendicular plane and 7 degrees in the parallel plane, said angles varying slightly according to different manufacturers' diode module construction. A lens is positioned in front of each diode at or near the point of exit of the laser beam to columnate said laser beam so as to control ils divergence from its point of exit.

Another feature of this invention is its two rows of teeth which are provided in this device so that the aforesaid function of parting the hair is conveniently effected whether the device is moved in either a forward or backward direction across the skin.

This invention has been described by way of example only and it should be appreciated that modifications and improvements may be made For example, the above description of the invention refers to a preferred laser diode frequency but other frequencies such as the infrared wavelength may be use. Also, in addition to the comb-like configuration described in the foregoing (where the row of lasers and combs are parallel to the body of the device) other configurations such as a rake-like one (where the row of lasers and teeth are at right-angles to the body of the device) are possible. Modification of the device from mains power to battery power usage is also possible.

## Claims

1. A hand-held laser skin treatment device for irradiating hair-growing skin comprising a casing, a laser assembly within the casing, the assembly including a radiation source and a power source for the radiation source, the radiation source consisting of a row of laser diodes, each diode having a lens and emitting a laser beam whereby the row of laser diodes emits a row of laser beams, and two parallel rows of teeth for parting hair, wherein the row of laser beams is centred between the parallel rows of teeth such that each beam is arranged between and associated with a pair of teeth, one from each of said rows of teeth, such that, in use when the device is moved transverse to the rows of teeth and beams in either direction across hair-growing skin, each beam is preceeded by one of the associated teeth which parts the hair to expose the skin in advance of the laser beam.

2. The device of claim 1 wherein the laser beams are visible red beams of wavelength in the range 632nm-670nm.

## Patentansprüche

1. Handgerät zur Laserhautbehandlung, um mit Haaren bewachsene Haut zu bestrahlen, das Folgendes umfasst: ein Gehäuse, eine Laserbaugruppe in dem Gehäuse, wobei die Baugruppe eine Strahlungsquelle und eine Energiequelle für die Strahlungsquelle beinhaltet, wobei die Strahlungsquelle aus einer Reihe von Laserdioden besteht, wobei jede Diode eine Linse aufweist und einen Laserstrahl emittiert, so dass die Reihe von Laserdioden eine Reihe von Laserstrahlen emittiert, und zwei parallele Reihen von zinken zum Teilen von Haar, wobei die Laserstrahlenreihe zwischen den parallelen Zinkenreihen zentriert ist, so dass jeder Strahl zwischen einem Paar Zinken und damit assoziiert angeordnet ist, einem von jeder der genannten Zinkenreihen, so dass beim Gebrauch, wenn das Gerät transversal zu den Zinkenreihen bewegt wird und in beiden Richtungen über die mit Haaren bewachsene Haut strahlt, jedem Strahl einer der assoziierten Zinken vorausgeht, die das Haar teilen, um die Haut vor dem Laserstrahl freizulegen.

2. Gerät nach Anspruch 1, wobei die Laserstrahlen sichtbare Rotstrahlen mit einer Wellenlänge im Bereich von 632 nm bis 670 nm sind.

## Revendications

1. Appareil laser de traitement dermique à main pour irradier une peau à croissance pileuse, comprenant un boîtier, un ensemble laser dans le boîtier, l'ensemble comprenant une source de rayonnement et une source de puissance pour la source de rayonnement, la source de rayonnement consistant en une rangée de diodes laser, chaque diode ayant une lentille et émettant un rayon laser en vertu de quoi la rangée de diodes laser émet une rangée de rayons laser, et deux rangées parallèles de dents pour séparer les poils, dans lequel la rangée de rayons laser est centrée entre les rangées parallèles de dents de telle sorte que chaque rayon est arrangé entre et associé à une paire de dents, une de chacune desdites rangées de dents, de telle sorte que pendant l'emploi lorsque l'appareil est déplacé à la transversale par rapport aux rangées de dents et de rayons dans chaque direction sur la peau à croissance pileuse, chaque rayon est précédé par l'une des dents associées qui sépare les poils pour exposer la peau devant le rayon laser.

2. L'appareil de la revendication 1, dans lequel les rayons laser sont des rayons rouges visibles d'une longueur d'onde dans la plage de 632 nm - 670 nm.
